# EUROPEAN PATENT SPECIFICATION

(11) **EP 0 573 466 B1**
(45) Date of publication and mention of the grant of the patent: **11.10.1995**
(21) Application number: 92904858.5
(22) Date of filing: 21.02.1992
(51) Int. Cl.: A61K 31/70, A23L 1/304, A23L 1/305, A23L 1/09, A23L 2/70, A23G 3/00, A23L 1/06

(54) **DIETETIC OR PHARMACEUTICAL COMPOSITIONS FOR THE RESTORATION OF ADENINE NUCLEOTIDE CELL CONTENT IN SKELETAL AND CARDIAC MUSCLES**
DIÄTETISCHE ODER PHARMAZEUTISCHE ZUSAMMENSETZUNGEN ZUR WIEDERHERSTELLUNG VON ADENINNUKLEOTIDZELLGEHALT IN SKELETT- UND HERZMUSKEL
COMPOSITIONS DIETETIQUES OU PHARMACEUTIQUES DESTINEES A RESTORER LA TENEUR DES CELLULES EN ACIDE ADENYLIQUE AU NIVEAU DES MUSCLES SQUELETTIQUES ET CARDIAQUES

(30) Priority: 01.03.1991 IT MI910553
(43) Date of publication of application: 15.12.1993
(73) Proprietor: DEPHA TEAM S.r.l., I-20090 Segrate (Milano) (IT)
(72) Inventor: PALAZZI, Camillo, Maria, Francesco, Giulio, Palazzo Tiepolo I-20090 Segrate (IT); PROCIDA, Carla Via Cassanese, 224, I-20090 Segrate (IT); RONCHI, Celestino Via Cassanese, 224, I-20090 Segrate (IT); CESCHEL, Giancarlo Via Cassanese, 224, I-20090 Segrate (IT)
(74) Representative: Minoja, Fabrizio
(86) International application number: EP9200369
(87) International publication number: WO9215311

(56) References cited:
- EP-A- 0 324 227
- FR-A- 2 609 397
- US-A- 4 824 660

## Description

The present invention relates to dietetic or pharmaceutical compositions containing a mixture of (D)-ribose and magnesium (L)-aspartate in various ratios.

One of the most important problems in the skeletal and cardiac muscle physiopathology is to restore, or to maintain adenine nucleotide cell content within physiological limits during or after a prolonged and/or exhausting physical effort, by a necessary or advisable nutritional intervention. On the contrary, the balance between energy requirement and energy availability would be jeopardized. This unfavourable situation occurs when dephosphorylation of ATP into ADP, and subsequently into AMP, continues till adenosine, inosine and ipoxanthine production.

These products are released by the cell (R.M. Berne; Am. J. Physiol., 204,317,1963), and therefore are lost for the purpose of a possible restoration of adenine nucleotides.

Theoretically, the problem of adenine nucleotide degradation could be solved by means of some biochemical-nutritional possibilities, for example, administration of adenosine (K. Reibel and M.J. Rovetto; Am. J. Physiol., 237,247,1979) or inosine (V.T. Wiedmeier, R. Rubio and R.M. Berne; J. Mol. Cell. Card.; 4,445,1972), which however proved to be rather uneffective.

It has now been found that the administration of (D)-ribose and magnesium (L)-aspartate surprisingly enhances adenine nucleotide synthesis, thus resulting in a higher availability of 5-phosphoribosyl-1-pyrophospate, which is the limiting factor of adenine nucleotide biosynthesis. Accordingly, administration of (D)-ribose prevents and reduces adenine nucleotide decrease in muscles during strong stress conditions.

Therefore, dietetic or pharmaceutical compositions containing (D)-ribose combined with magnesium (L)-aspartate are an object of the present invention.

In fact, it is well known that about 55% magnesium ions in the human body are located in the bones, while the remaining are in the soft tissues. A decrease in muscle Mg²⁺ has particularly been observed during magnesium deficiency and during intense physical exercises.

Magnesium ion mobilization, particularly the loss thereof from muscle cells during physical exercise, can be explained by the high activity of the Mg²⁺ dependent enzymes, which are involved in the energy metabolism (creatinine phosphokinase, glycogen phosphorylase and myosin ATPase). The correlation between Mg²⁺ loss and exercise intensity can depend on: i) a reduced kidney concentrating capacity which is induced directly by the physical exercise or indirectly by the increase of the hormones inducing Mg²⁺ ion tubular reabsorption (aldosterone, antidiuretic hormone, thyroid hormones) whose hematic concentration can remain high up to 14 hours after the end of the physical exercise; ii) acidosis, due to lactate accumulation, which can induce magnesiuria through a decrease in magnesium tubular reabsorption.

Accordingly, the (D)-ribose and magnesium (L)-aspartate combination assures a correct adenine nucleotide increase and, at the same time, allows to balance muscle Mg²⁺ concentration both in magnesium deficiencies and during physical exercises. Administration of the compositions of the present invention is also useful in pathologic conditions wherein a prompt restoration of weary muscles is necessary, for example in diabetes, alcoholism, cardiopathies, pregnancy.

The dietetic compositions of the present invention contain from 200 to 2000 mg of (D)-ribose. The magnesium (L)-aspartate unitary dose can range from 100 to 1000 mg. Weight ratios of (D)-ribose to magnesium (L)-aspartate are not critical and will generally range from 1:1 to 5:1.

The compositions of the present invention can further contain other active ingredients or integrators with adjuvant, complementary or useful activities.

Examples of said elements which are profitably used are:
- mineral salts and/or vitamins
- potassium aspartate.

The dietetic or pharmaceutical compositions of the invention can be prepared according to conventional techniques and excipients. Said compositions are prepared by admixing (D)-ribose and magnesium (L)-aspartate with physiologically acceptable excipients having pleasant appearance, smell and taste.

Examples of excipients known in the food industry are diluents, sweeteners, binders, flavoring aids, lubricants and non-sticking agents, natural and artificial food dyes.

Examples of diluents are: microcrystalline cellulose, glycine, lactose; maize, patatoes and rice starch; mannitol, sorbitol, sucrose, fructose; examples of sweeteners are: saccharin sodium, saccharin acid, aspartame, honey; examples of binders are: starch, polyvinylpyrrolidone, polyvinyl alcohol, hydroxymethylcellulose; examples of flavor aids are: citric acid and the salts thereof, tartaric acid and the salts thereof, sodium glutamate, sodium chloride, orthophosphoric acid and the salts thereof, menthol; examples of lubricants and non-sticking agents are: magnesium stearate, talc, 200 to 6000 PEGs (polyethylene glycols), glyceryl behenate, glycerin, mineral oil, silicone oils, levynite; examples of food dyes are: chlorophyll, bilberry anthocyanins; E104, E110; titanium dioxide, iron oxides; examples of dietetic compositions are: chewable, effervescent or swallowable tablets, syrups, fruit beverages, soluble granulate sachets, fruit jellies, candies.

The following examples further illustrate the invention.

### EXAMPLE 1

### Chewable tablets

| 1 Tablet contains | |
|---|---|
| (D)-Ribose | mg 200 |
| Magnesium (L)-aspartate | mg 200 |
| Maize starch | mg 50 |
| Lactose | mg 50 |
| Magnesium stearate | mg 5 |

### EXAMPLE 2

### Drinkable solution

| 250 ml of solution contain: | |
|---|---|
| (D)-Ribose | mg 1000 |
| Magnesium (L)-aspartate | mg 1000 |
| Sorbitol | mg 30 |
| Citric acid | mg 50 |
| Sodium chloride | mg 50 |
| Methyl-p-hydroxybenzoate | mg 45 |
| Propyl-p-hydroxybenzoate | mg 5 |
| Purified water q.s. to | ml 250 |

### EXAMPLE 3

### Granulate sachet

| 1 Sachet contains: | |
|---|---|
| (D)-Ribose | mg 1000 |
| Magnesium (L)-aspartate | mg 500 |
| Lactose | mg 200 |
| Methylcellulose | mg 10 |
| Tartaric acid | mg 5 |
| Lemon flavor | mg 25 |
| Sorbitol q.s. to | g 3 |

### EXAMPLE 4

### Chewable tablets

| 1 Tablet contains: | |
|---|---|
| (D)-Ribose | mg 500 |
| Magnesium (L)-aspartate | mg 500 |
| Mannitol q.s. to | g 1,5 |
| Polyvinylpyrrolidone | mg 50 |
| Citric acid | mg 10 |
| Sodium chloride | mg 3 |
| Orange flavor | mg 20 |
| Magnesium stearate | mg 10 |
| Talc | mg 8 |

### EXAMPLE 5

### Sugar pills

| 1 Sugar pill contains: | |
|---|---|
| (D)-Ribose | mg 500 |
| Magnesium (L)-aspartate | mg 100 |
| Maize starch | mg 50 |
| Talc | mg 30 |
| Levylite | mg 25 |
| Magnesium stearate | mg 5 |
| Sucrose | mg 150 |
| E110 | mg 0,025 |
| Carnauba wax | mg 0,001 |

## Claims

1. Dietetic or pharmaceutical compositions containing a mixture of from 200 to 2000 mg of (D)-ribose and of magnesium (L)-aspartate as active ingredient.

2. Dietetic or pharmaceutical compositions according to claim 1 containing from 100 to 1000 mg of magnesium (L)-aspartate.

3. Dietetic or pharmaceutical compositions according to anyone of the preceding claims, in the form of effervescent, chewable and swallowable tablets, syrups, fruit drinks, soluble granulate sachets, fruit jellies, candies.

## Patentansprüche

1. Diätetische oder pharmazeutische Zusammensetzungen, die eine Mischung von 200 bis 2000 mg (D)-Ribose und Magnesium (L)-Aspartat als Wirkstoff enthalten.

2. Diätetische oder pharmazeutische Zusammensetzungen nach Anspruch 1, die von 100 bis 1000 mg Magnesium (L)-Aspartat enthalten.

3. Diätetische oder pharmazeutische Zusammensetzungen nach einem der vorherigen Ansprüche, in Form von aufbrausenden, kaubaren und zum Schlucken Tabletten, Sirupen, Getränken mit Obstgeschmack, löslichen granulierten Beutelchen, Gelatinen mit Obstgeschmack, Bonbons.

## Revendications

1. Compositions diététiques ou pharmaceutiques contenant un mélange de 200 à 2000 mg de (D)-ribose et de magnésium (L)-aspartate comme principe actif.

2. Compositions diététiques ou pharmaceutiques selon la revendication 1, contenant de 100 à 1000 mg de magnésium (L)-aspartate.

3. Compositions diététiques ou pharmaceutiques selon l'une quelconque des revendications précédentes, sous forme de comprimés effervescents, qu'on peut mâcher et à avaler, sirops, boissons à base de fruits, sachets de grené soluble, gelées à base de fruits, bonbons.
